Europäisches Patentamt

European Patent Office

Office européen des brevets

(19)

(11) **EP 1 119 344 B1**

(12) **FASCICULE DE BREVET EUROPEEN**

(45) Date de publication et mention
de la délivrance du brevet:
**22.12.2004 Bulletin 2004/52**

(21) Numéro de dépôt: **99970017.2**

(22) Date de dépôt: **05.10.1999**

(51) Int Cl.⁷: **A61K 7/48**

(86) Numéro de dépôt international:
**PCT/FR1999/002375**

(87) Numéro de publication internationale:
**WO 2000/019974 (13.04.2000 Gazette 2000/15)**

(54) **METHODE DE PREVENTION ET/OU DE TRAITEMENT COSMETIQUE DES VERGETURES DE LA PEAU ET UTILISATION EN DERMATOLOGIE**

**METHODE ZUR PRÄVENTION UND BEHANDLUNG VON DEHNUNGSSTREIFEN (STRIAE DISTENSAE) UND DEREN VERWENDUNG IN DERMATOLOGIE**

**METHOD FOR PREVENTION AND/OR COSMETIC TREATMENT OF SKIN STRIPE AND USE IN DERMATOLOGY**

(84) Etats contractants désignés:
**AT BE CH CY DE DK ES FI FR GB GR IE IT LI LU MC NL PT SE**

(30) Priorité: **05.10.1998 FR 9812435**

(43) Date de publication de la demande:
**01.08.2001 Bulletin 2001/31**

(73) Titulaire: **Laboratoires Expanscience
92419 Courbevoie Cedex (FR)**

(72) Inventeur: **MSIKA, Philippe
F-75018 Paris (FR)**

(74) Mandataire: **Warcoin, Jacques
Cabinet Régimbeau
20, rue de Chazelles
75847 Paris cedex 17 (FR)**

(56) Documents cités:
**US-A- 5 444 091          US-A- 5 759 555**

## Description

La présente invention concerne une méthode de prévention et/ou de traitement cosmétique des vergetures de la peau et l'utilisation d'une composition pour la préparation d'un médicament de dermatologie destiné à la prévention et/ou au traitement des vergetures.

Les vergetures sont des marques visibles sur la peau résultant de l'étirement de cette dernière du fait d'un gain de poids, de contraintes mécaniques, qui concernent le plus souvent les femmes, après la puberté ou une première grossesse. Environ 50% de femmes enceintes vont développer des vergetures, sur les cuisses, l'abdomen et/ou les seins. Les vergetures peuvent également apparaître lors d'états physiologiques ou pathologiques tels que l'obésité, la tuberculose et la fièvre thyphoïde, et également lors d'un régime alimentaire relativement intensif.

Le traitement des vergetures a été décrit par exemple dans l'article de P. Zheng et al, "Anatomy of striae", British Journal of Dermatology 112:185-193 (1985), dans lequel notamment on rapporte que les vergetures sont des cicatrices résultant d'un processus d'inflammation détruisant les fibres élastiques.

Depuis, divers composés ont été proposés en tant que principes actifs pour traiter les vergetures, tels que par exemple la trétinoine (ou all-trans acide rétinoïque). D'après l'article de R.E.B. Watson et al, "Fibrillin microfibrils are reduced in skin exhibiting striae distensae", British Journal of Dermatology 138: 931-937 (1998) il semblerait que la trétinoine ait une action anti-vergetures tendant à restaurer essentiellement le réseau des fibrillines, qui sont le principal constituant des microfibrilles comprises dans les fibres élastiques, par rapport à d' autres constituants de la matrice extracellulaire.

Toutefois, si les composés actifs de l'art antérieur permettent d'obtenir un effet de régression des vergetures, il n'en reste pas moins que les résultats obtenus ne sont pas totalement satisfaisants, en particulier compte tenu du problème d'intolérance cutanée bien connu de la trétinoine. Il existait donc jusqu'à ce jour une réelle demande pour l'élaboration d'un produit permettant de prévenir et/ou de traiter efficacement, avec une tolérance cutanée acceptable, ce phénomène complexe et particulièrement inesthétique que sont les vergetures.

On a maintenant trouvé de manière tout à fait surprenante et inattendue que l'utilisation de certains peptides permet de prévenir et/ou de traiter de manière tout à fait significative, et acceptable quant à la tolérance cutanée, les vergetures de la peau.

La présente invention a ainsi pour objet une méthode de prévention et/ou de traitement cosmétique des vergetures de la peau, caractérisée par le fait que l'on applique sur les zones de la peau susceptibles de former ou comprenant des vergetures une composition comprenant, dans un véhicule approprié, au moins un agent anti-vergetures choisi dans le groupe constitué par les peptides du soja, les tripeptides constitués des acides aminés Glycine, Histidine et Lysine, et les mélanges de ces peptides.

Par l'expression "prevention des vergetures de la peau", on entend selon la présente invention une action permettant d'éviter ou à tout le moins de réduire la formation de vergetures, c'est-à-dire leur longueur, largeur et/ou profondeur, dans le cadre d'un traitement cosmétique ou dermatologique, par application de la composition, avant et au cours d'un évènement connu comme pouvant provoquer l'apparition de vergetures, tel qu'une grossesse. Par l'expression "traitement des vergetures de la peau", on entend selon la présente invention une action permettant de faire régresser, c'est-à-dire de résorber, dans le cadre d'un traitement cosmétique ou dermatologique, de manière visible et mesurable, des vergetures déjà formées, c'est-à-dire leur longueur, largeur et/ou profondeur.

Ainsi, la composition utilisée selon l'invention peut être appliquée sur des zones de la peau susceptibles de former des vergetures, comprenant des vergetures en cours de formation ou même comprenant des vergetures déjà formées

Les peptides du soja dans la composition utilisée selon la présente invention peuvent être tout peptide obtenu par hydrolyse de protéines extraites du soja, selon des conditions opératoires connues dé l'homme du métier, en d'autres termes tout hydrolysat de protéine du soja. De préférence, ces peptides du soja sont des peptides ayant subi en outre une fermentation par une souche de micro-organisme. D'une manière générale, on obtient un peptide du soja fermenté en plaçant un peptide du soja dans un fermenteur en présence de glucose, de sels minéraux et d'une souche de micro-organisme donnée, dans des conditions contrôlées de température, de pH, d'oxygénation et de durée. Après la fermentation, on obtient le peptide du soja fermenté par des opérations de séparation et de filtration classiques. Cette technique est notamment mise en oeuvre par la société COLETICA qui commercialise ainsi divers hydrolysats de protéines végétales fermentés. De préférence, les peptides du soja, fermentés ou non, dans la composition utilisée selon la présente invention, ont un poids moléculaire compris entre environ 200 et environ 20000 Daltons, tel que mesuré par exemple par électrophorèse.

Un peptide du soja particulièrement préféré pour la composition utilisée selon l'invention est le peptide fermenté dénommé "Phytokine ®" tel que commercialisé par la société COLETICA.

Ce peptide du soja fermenté spécifique, de poids moléculaire moyen d'environ 800 Daltons, est obtenu par fermentation d'un peptide du soja par la souche de micro-organisme Lactobaccillus et son aminogramme est le suivant :

|  | Nombre de résidus pour 100 |
|---|---|
| Hyp | 0,39 |
| Asp | 12,64 |
| Thr | 2,93 |
| Ser | 4,29 |
| Glu | 20,08 |
| Pro | 7,31 |
| Gly | 7,95 |
| Ala | 7,76 |
| Cys | ND* |
| Val | 5,59 |
| Met | 0,96 |
| Ile | 4,46 |
| Leu | 7,42 |
| Tyr | 1,38 |
| Phe | 3,39 |
| His | 2,12 |
| Hyl | 0,09 |
| Lys | 5,73 |
| Trp | ND* |
| Arg | 5,53 |
| βAla | ND |

(*ND : non déterminé)

Par "tripeptide constitués des acides aminés Glycine, Histidine et Lysine", on entend en particulier les tripeptides de séquence Gly-His-Lys, dont les acides aminés peuvent être sous la forme D, L ou DL, éventuellement conjugués avec un acide carboxylique tel que l'acide acétique, sous forme d'un complexe avec un métal tel que le zinc ou le cuivre.

Parmi les tripeptides constitués des acides aminés Glycine, Histidine et Lysine, on préfère utiliser le tripeptide "KOLLAREN-CPP" dont la dénomination INCI est "tripeptide-1", tel que commercialisé par la société SEPORGA. Le "KOLLAREN-CPP" est un tripeptide de séquence Gly-His-Lys conjuguée avec l'acide acétique (acétate), sous forme d'un complexe avec du zinc.

Ainsi, plus particulièrement, la présente invention concerne une méthode de prévention et/ou de traitement cosmétique des vergetures de la peau, caractérisée par le fait que l'agent anti-vergetures est choisi dans le groupe constitué par le peptide Phytokine® du soja, le tripeptide KOLLAREN-CPP® et les mélanges de ces peptides.

Dans la composition utilisée selon l'invention, la proportion en agent anti-vergetures est comprise entre environ 0,1 et environ 10 % en poids, par rapport au poids total de la composition.

Selon un mode de réalisation préféré, la composition utilisée selon la présente invention comprend en outre au moins un α-hydroxy acide, en association avec l'agent anti-vergetures. On a en effet constaté de manière surprenante que l'utilisation conjointe d'un α-hydroxy acide permet au moins de potentialiser l'activité de l'agent anti-vergetures sinon, dans certains cas, d'obtenir un effet de synergie pour la prévention et/ou le traitement des vergetures.

L'α-hydroxy acide utilisé selon l'invention peut être tout α-hydroxy acide permettant d'obtenir un effet d'exfoliation et/ou d'hydratation de la peau, tels que, par exemple, l'acide citrique, l'acide piruvique, l'acide glycolique ou encore l'acide lactique.

Un α-hydroxy acide particulièrement préféré pour la composition utilisée selon l'invention est l'acide lactique.

La proportion en α-hydroxy acide est de préférence comprise entre environ 0,1 et environ 20 % en poids, par rapport au poids total de la composition.

De préférence, la composition utilisée selon l'invention comprend un agent anti-vergetures choisi dans le groupe constitué par le peptide Phytokine® du soja, le tripeptide KOLLAREN-CPP® et les mélanges de ces peptides, en association avec l'acide lactique en tant qu'α-hydroxy acide. On a en effet remarqué qu'une telle association permet de fournir un effet particulièrement avantageux concernant l'activité anti-vergetures de la composition utilisée selon l'invention.

Enfin, la composition utilisée selon l'invention comprend avantageusement en outre un composé destiné à régler le pH de la composition selon l'invention, à une valeur comprise entre environ 2 et environ 4, et de préférence une valeur d'environ 3,5, notamment pour neutraliser partiellement l'α-hydroxy acide. En particulier, on peut utiliser l'arginine ou une alcanolamine telle que la triéthanolamine.

Selon un mode de réalisation particulièrement préféré, la composition utilisée selon l'invention comprend en outre un composé inhibiteur de substance P et du neuropeptide Y (ou ci-après NPY). Ce composé additionnel peut être choisi parmi les composés inhibiteurs de la substance P et du NPY connus de l'homme du métier.

Toutefois, un composé inhibiteur de substance P et du NPY particulièrement préféré est un extrait spécifique comprenant une fraction de peptides actifs, obtenu à partir de l'algue verte (ou chlorophycée) dénommée "*Enteromorpha compressa*" (ou "Ao-nori" ou encore " yellow green nori " ) tel que celui commercialisé par la société SECMA sous la dénomination "Enteline 2" (dénomination INCI "butylene glycol, glycérine, *Enteromorpha compressa extract* ; n° CAS 92128-82-0).

On a en effet observé que l'utilisation de ce composé spécifique inhibiteur de la substance P et du neuropeptide Y permet d'obtenir un effet particulièrement avantageux de tolérance de la composition utilisée selon l'invention, en particulier compte tenu des effets irritants de l'α-hydroxy acide notamment de l'acide lactique

La proportion en composé inhibiteur de la substance P et du NPY dans la composition utilisée selon l'invention est de préférence comprise entre environ 0,1 et environ 5 % en poids, par rapport au poids total de la composition.

De préférence, la composition utilisé selon l'invention comprend un agent anti-vergetures choisi dans le groupe constitué par le peptide Phytokine® du soja, le tripeptide KOLLAREN-CPP® et les mélanges de ces peptides, en association avec l'acide lactique et l'extrait Enteromorpha compressa.

La composition utilisée selon l'invention comprend également un véhicule approprié qui peut être tout véhicule parmi ceux connus de l'homme du métier en vue d'obtenir une composition cosmétique ou dermatologique utilisable selon l'invention, sous forme d'une crème, d'une lotion, d'un gel, d'une pommade, etc. éventuellement sous la forme d'une émulsion, avec en outre des composants connus de l'homme du métier pour améliorer, modifier ou stabiliser la composition d'un point de vue cosmétique ou dermatologique.

En particulier, la composition utilisée selon l'invention peut en outre comprendre des composés contribuant de manière secondaire à l'action anti-vergetures, tels que l'extrait de *Sophora japonica*, qui contribue à un contrôle de la vascularisation des vergetures et donc de la couleur de ces dernières, ou encore des composés à silanols tels que le lactacte méthylsilanol ou des oligoéléments à base de cuivre et zinc constitutifs des protéines du derme, tels que le gluconate de zinc et le gluconate de cuivre.

Les conditions opératoires pour préparer la composition utilisée selon l'invention font parties des connaissances générales de l'homme du métier.

Enfin, la présente invention a également pour objet l'utilisation d'une composition telle que définie ci-dessus pour la préparation d'un médicament de dermatologie destiné à la prévention et/ou au traitement des vergetures de la peau.

Les exemples suivants sont destinés à illustrer la présente invention et ne doivent en aucun cas être interprétés comme pouvant en restreindre la portée.

**Exemple 1 : crème anti-vergetures ayant un pH de 3,5.**

| | % |
|---|---|
| - Cétyl diméthcone, commercialisé sous la dénomination "Albilwax 9801" par la société Goldschmitt | 2 |
| - Sébacate d'octyle | 5 |
| - Isononanoate d'Isononyle | 7 |
| - Mélange de stéarate de glycéryle, d'alcool cétéarylique, de palmitate de cétyle et de cocoglycérides, commercialisé sous la dénomination "Cutina CBS" par la société Sidobre Sinnova | 2,5 |
| - Méthylparaben | 0,1 |
| - Propylparaben | 0,1 |
| - Eau | qsp 100 |
| - PEG 300 | 5 |
| - Triéthanolamine | 4,8 |
| - Sepigel 305® (épaississant commercialisé par la société SEPPIC) | 5,5 |
| - Phytokine® | 2 |
| - Acide lactique | 10 |
| - Enteline 2 ® | 0,4 |

(suite)

|  | % |
|---|---|
| - Sophora Japonica | 3 |
| - Lactate Méthylsilanol | 3 |
| - Gluconate de zinc | 0,2 |
| - Gluconate de cuivre | 0,2 |
| - Parfum | 0,35 |

**Exemple 2 : crème anti-vergetures ayant un pH de 3,5**

|  | % |
|---|---|
| - Cétyl diméthcone, commercialisé sous la dénomination "Albilwax 9801" par la société Goldschmitt | 2 |
| - Sébacate d'octyle | 5 |
| - Isononanoate d'Isononyle | 7 |
| - Mélange de stéarate de glycéryle, d'alcool cétéarylique, de palmitate de cétyle et de cocoglycérides, commercialisé sous la dénomination "Cutina CBS" par la société Sidobre Sinnova | 2,5 |
| - Méthylaraben | 0,1 |
| - Propylparaben | 0,1 |
| - Eau | qsp 100 |
| - PEG 300 | 5 |
| - Triéthanolamine | 4,8 |
| - Sepigel 305® (épaississant commercialisé par la société SEPPIC) | 5,5 |
| - Kollaren CPP® | 2,5 |
| - Acide lactique | 10 |
| - Enteline 2® | 0,4 |
| - Sophora Japonica | 3 |
| - Lactate Méthylsilanol | 3 |
| - Gluconate de zinc | 0,2 |
| - Gluconate de cuivre | 0,2 |
| - Parfum | 0,35 |

**Exemple 3 : Etude clinique pour l'évaluation de l'effet des compositions des exemples 1 et 2, sur la régression des vergetures, sur la base d'une évaluation instrumentale associée a une évaluation clinique, après applications cutanées répétées, dans les conditions normales d'utilisation, pendant 6 semaines, chez 9 volontaires adultes de sexe feminin.**

1. Objectif de l'étude

La présente étude a pour objet d'évaluer et de comparer l'effet sur la "régression" des vergetures des compositions des exemples 1 et 2 ci-dessus, par des mesures colorimétriques des vergetures de la peau des cuisses, associées à des mesures des paramètres biomécaniques et à une évaluation clinique, après des applications cutanées répétées pendant 6 semaines, dans les conditions normales d'utilisation, chez 9 volontaires adultes de sexe féminin.

2. Pertinence de l'essai

La mesure des paramètres viscoélastiques cutanés à l'aide d'un Cutomètre® permet de déterminer l'effet d'un produit sur les propriétés biomécaniques de la peau, après des applications répétées. Cet appareil mesure la déformation d'une zone cutanée, soumise à une contrainte mécanique de succion et son pouvoir de récupération (Wilhelm et al., 1993). Les propriétés viscoélastiques de la peau sont en effet corrélées aux notions de souplesse, d'élasticité et de fermeté du tégument.

Les mesures à l'aide d'un Chromamètre permettent, par ailleurs, d'évaluer objectivement l'effet d'un produit sur la coloration de la peau, au niveau d'une zone présentant des vergetures, en comparaison à une zone témoin (peau normale).

Associées à une évaluation clinique sur la base de scores par le Directeur de l'Etude, ces techniques instrumentales permettent d'évaluer l'effet d'un produit sur les vergetures, chez un panel de 9 volontaires adultes de sexe féminin, après 6 semaines d'applications biquotidiennes, dans les conditions normales d'utilisation.

3. Volontaires

9 panélistes ont été définitivement admis par le Directeur de l'Etude sur la base d'un examen clinique spécifique à l'étude, réalisé juste avant le début de l'essai. Ils ont tous participé à la totalité de l'essai.

L'analyse des résultats a donc porté sur un panel de 9 volontaires adultes de sexe féminin (ou 8 pour les mesures colorimétriques), âgés de 20 à 31 ans (moyenne d'âge: 26 ans), présentant des vergetures datant de moins de 8 mois.

4. Protocole

4.1 Evaluations instrumentales et cliniques initiales:

4.1.1. Détermination des paramètres viscoélastiques:

Ces paramètres ont été évalués au niveau de la peau des 2 cuisses à l'aide du Cutomètre™ (Courage + Khazaka, Allemagne), sur deux zones diamétralement opposées, délimitées au niveau des cuisses droite et gauche de chacun des volontaires adultes de sexe féminin, spécifiquement sélectionnés et recrutés pour la réalisation et l'objectivation de ce type d'essai. Ces mesures ont été réalisées au niveau d'une zone présentant une vergeture, ainsi qu'au niveau d'une zone adjacente, sans vergeture ("peau normale"), après repérage des zones au moyen d'un cache en plastique transparent muni de repères anatomiques.

4.1.2. Mesure de la coloration

L'évaluation de la coloration d'une vergeture a été effectuée au niveau de la peau des deux cuisses et d'une zone témoin adjacente (sans vergeture) par l'analyse de la variable de clarté "L*" et des coordonnées de chromaticité "a*" et "b*", à l'aide du Chromamètre CR 321 (Minolta) muni d'un cône permettant des mesures colorimétriques sur une surface de 3 mm de diamètre.

Ces mesures ont été réalisées après une période de repos de 20 minutes environ, dans une pièce climatisée où la température ambiante a été maintenue à $22 \pm 2°$ C et l'humidité relative à $50 \pm 5$ %, grâce à un microprocesseur relié à des capteurs-transmetteurs de température et d'humidité, afin d'atteindre un équilibre stable d'échange d'eau entre la peau de chaque panéliste et le milieu environnant. La stabilité de ces paramètres a été contrôlée et imprimée en continu à l'aide d'un enregistreur multivoies.

4.1.3. Evaluation Clinique

Les critères de jugement suivants ont été évalués, par le Directeur d'Etude Adjoint, sur la base de scores cliniques en 9 points (1 à 9), au niveau des deux cuisses, pour chacun des volontaires:

. taille des vergetures,
. couleur des vergetures,
. relief des vergetures.

4.1.4. Photographies

Des macrophotographies en couleur ont été prises au niveau d'une zone cutanée de chaque cuisse, à l'aide d'un appareil Nikon F-801S, muni d'un objectif macro Nikon 105MM, sous un éclairage de type "lumière du jour" (6500° K).

4.2. Détermination de l'efficacité des produits, après des applications répétées

4.2.1. Modalités des applications

Les produits étudiés ont été appliqués deux fois par jour, pendant 6 semaines consécutives, dans les conditions normales d'utilisation, par le volontaire lui-même à son domicile, sur la peau des cuisses (1 produit pour chaque cuisse selon une randomisation - loi binomiale).

Afin de standardiser au maximum les conditions de l'étude, l'application des produits étudiés a été effectuée une fois par semaine, en présence du personnel du laboratoire.

4.2.2. Effet sur les propriétés viscoélastiques de la peau (tonicité, fermeté, souplesse, élasticité)

Les paramètres viscoélastiques de la peau des 2 cuisses (zones avec et sans vergetures), repérées avec précision par rapport au premier jour de l'essai et selon le même principe, ont été déterminés après la sixième semaine d'utilisation des produits. Cette évaluation a été réalisée 16 à 24 heures après la dernière application des produits, par le personnel du laboratoire, de manière à mesurer spécifiquement les variations des paramétres élastiques du tissu cutané induites par les utilisations répétées.

4.2.3. Effet sur la coloration de la peau

Les mesures de la coloration de la peau ont été réalisées, à l'aide du Chromamètre®, après les 6 semaines d'utilisation, au niveau des zones déterminées lors du premier jour de l'étude et exactement repérées, selon le même principe (zones avec et sans vergetures).

4.2.4. Evaluations cliniques et auto-évaluations

Les évaluations de la peau des deux cuisses ont été effectuées par le Directeur de l'Etude, sur la base de scores cliniques en 6 points, selon le même principe que celui suivi lors de la détermination initiale, après les 6 semaines d'applications.

4.2.5. Photographies

Des macrophotographies en couleur ont été prises, au niveau des zones déterminées lors du premier jour de l'étude et exactement repérées, selon le même principe, après les 6 semaines d'applications.

4.3. Analyse et interprétation des résultats

4.3.1. Paramètres biomécaniques

- Les valeurs moyennes des paramètres viscoélastiques déterminés à J1 et J43 au niveau des 2 cuisses (zones avec vergetures et zones sans vergeture), ont été calculées par la détermination de la moyenne arithmétique et de l'écart obtenu par rapport à la moyenne (S.E.M.) des mesures individuelles effectuées sur l'ensemble des panélistes.
- Les valeurs initiales obtenues sur les cuisses droite et gauche (avant la première application des produits), ont été comparées par une analyse de variance (ANOVA, significativité: $p < 0,05$).
- Les valeurs obtenues après les 6 semaines d'utilisation des produits, ont été comparées aux valeurs initiales, déterminées avant la première application, par le test "t" de Student en séries appariées ("one-tail", significativité: $p < 0,05$), pour chacune des zones (cuisses droite et gauche, zones avec et sans vergetures).
- Les effets obtenus au niveau des cuisses droite et gauche (zones avec et sans vergetures) ont été comparés par une analyse de variance (ANOVA, significativité: $p < 0,05$) et par le test des comparaisons multiples ("L.S.D."), portant sur les différences calculées entre les valeurs acquises après les 6 semaines d'utilisation et les valeurs initiales ($\Delta$J43-J1).
- Les pourcentages de variation moyens des paramètres évalués au cours de l'essai, ont été calculés pour chaque zone cutanée, après les 6 semaines d'applications, par rapport à la valeur initiale, à partir des valeurs moyennes obtenues sur l'ensemble des panélistes.

4.3.2. Mesures colorimétriques

- Les valeurs moyennes des paramètres colorimétriques, déterminés à chaque temps de l'étude, ont été calculées par la détermination de la moyenne arithmétique et de l'écart par rapport à la moyenne (S.E.M.) des mesures individuelles effectuées sur l'ensemble des panélistes.

Ces déterminations portent sur la variable de clarté "L*", sur les coordonnées de chromaticité "a*" et "b*", ainsi que sur l'Angle Typologique Individuel ITA°, calculé selon la formule suivante:

$$ITA° = [ \text{arc tangente} (L* - 50) / b* ] \ 180 / 3.14159$$

- Les valeurs initiales obtenues sur les cuisses droite et gauche (avant la première application des produits) ont été comparées par une analyse de variance (ANOVA, significativité: $p < 0,05$).
- Les valeurs obtenues après les 6 semaines d'utilisation des produits, ont été comparées aux valeurs initiales, déterminées avant la première application, par le test "t" de Student en séries appariées ("one-tail", significativité: $p < 0,05$), pour chacune des zones (cuisses droite et gauche, zones avec et sans vergetures).
- Les effets obtenus au niveau des cuisses droite et gauche (zones avec et sans vergetures) ont été comparés par une analyse de variance (ANOVA, significativité: $p < 0,05$) et par le test des comparaisons multiples ("L.S.D."), portant sur les différences calculées entres les valeurs acquises après les 6 semaines d'utilisation et les valeurs initiales ($\Delta$J43-J1).
- Les pourcentages de variation moyens des paramètres évalués au cours de l'essai, ont été calculés pour chaque zone cutanée, après les 6 semaines d'applications, par rapport à la valeur initiale, à partir des valeurs moyennes obtenues sur l'ensemble des panélistes.

4.3.3. Scores cliniques

- Les valeurs moyennes des critères de jugement déterminés à chaque temps de l'étude sur la base des scores cliniques ont été calculées par la détermination de la moyenne arithmétique et de l'écart-type (Sd) des données individuelles acquises sur l'ensemble des panélistes.
- Les valeurs obtenues, après l'application des produits, ont été comparées aux valeurs déterminées lors du premier jour de l'essai (évaluations initiales), par le test de Wilcoxon en séries appariées ("one-tail" significativité: $p < 0,05$), pour chaque zone traitée.
- L'effet des produits a été comparé par un test de Wilcoxon en séries appariées ("one-tail", significativité: $p < 0,05$) portant sur les valeurs obtenues avant et après les applications répétées.
- Les pourcentages de variation moyens de chacun des critères d'évaluation, ont été calculés par rapport aux données initiales, à partir des valeurs moyennes obtenues sur l'ensemble des volontaires.

5. Résultats et conclusion

5.1. Mesures cutométriques

L'analyse statistique a préalablement démontré que les valeurs initiales des paramètres biomécaniques étaient identiques, d'une part, sur chacune des zones sans vergeture et, d'autre part, sur chacune des zones avec vergetures. Des différences statistiquement significatives ont, par ailleurs, été mises en évidence entre les zones avec et sans vergeture(s), traduisant une peau plus relâchée et moins élastique au niveau des zones avec vergetures.

5.1.1. Crème anti-vergetures de l'exemple 1

L'analyse des résultats a permis de mettre en évidence, après 6 semaines d'applications, par rapport à la mesure initiale:

- une tendance à une diminution de Uf (élongation finale), de 4% environ lors de la 1 ère et de la 3ème contrainte,
- une diminution statistiquement significative de Uv/Ue (taux de viscoélasticité déterminant l'importance de la réponse visqueuse par rapport à la réponse élastique), de l'ordre de 14%.

- Au niveau de la zone avec vergetures:

. une tendance à une diminution de Uf (élongation finale), de l'ordre de 2% lors de la 1ère et de la 3ème contrainte,
. une stabilisation de Ua/Uf (taux de récupération après contrainte),
. une diminution statistiquement significative de Uv/Ue (taux de viscoélasticité déterminant l'importance de la réponse visqueuse par rapport à la réponse élastique) de l'ordre de -17%.

On constate ainsi une amélioration significative des composantes de la fermeté et de la tonicité, au niveau de la zone avec vergetures.

5.1.2. Crème anti-vergetures de l'exemple 2

L'analyse des résultats a permis de mettre en évidence, après 6 semaines d'applications, par rapport à la mesure initiale:

- Au niveau de la zone sans vergeture:

une diminution statistiquement significative de Uf (élongation finale), de l'ordre de 6% lors de la 1ère et de la 3ème contrainte,
. une stabilisation de Ua/Uf (taux de récupération après contrainte), une stabilisation de Uv/Ue (taux de viscoélasticité).

- Au niveau de la zone avec vergetures:

. une tendance à une diminution de Uf (élongation finale), de l'ordre de 2% lors de la 1ère contrainte,
. une stabilisation de Ua/Uf (taux de récupération après contrainte),
. une tendance à une diminution de Uv/Ue (taux de viscoélasticité), de 9% environ.

On constate ainsi une nette tendance (non significative sur les 9 panélistes) à l'amélioration des composantes de la tonicité et de la fermeté de la peau au niveau de la zone avec vergetures.

5.2. Mesures colorimétriques

L'analyse statistique a préalablement démontré que les valeurs initiales des paramètres colorimétriques étaient identiques, d'une part, sur chacune des zones sans vergeture et, d'autre part, sur chacune des zones avec vergetures. A noter que la peau des zones avec vergetures (avant et après les 6 semaines d'utilisation des produits) était plus claire que celle des zones sans vergeture (variable de clarté L* et I.T.A.° plus élevés).

Aucune modification favorable et statistiquement significative des paramètres colorimétriques n'a été enregistrée, après utilisation de chacun des produits, quelles que soient les zones (avec et sans vergeture(s)).

5.3. Evaluations cliniques par le Directeur de l'Etude

L'analyse des résultats a permis de mettre en évidence une amélioration statistiquement significative des critères suivants, à l'exception de la longueur des vergetures. Une différence significative a par ailleurs, été relevée entre les deux produits étudiés pour ce critère, traduisant une régression plus importante des vergetures au niveau de la zone traitée avec la Crème Anti-Vergetures de l'exemple 2.

| | Crème anti-vergetures exemple 1 | Crème anti-vergetures exemple 2 |
|---|---|---|
| Largeur des vergetures (fines->larges) | -17%* | - 14% |
| Longueur des vergetures (courtes->longues) | -8% | -14%• |

* valeur statistiquement significative au temps 6 semaines, par rapport à l'évatuation initiale

• diminution statistiquement significative en comparaison à la crème anti-vergetures de l'exemple 1 (test de Wilcoxon, "one-tail").

(suite)

| | Crème anti-vergetures exemple 1 | Crème anti-vergetures exemple 2 |
|---|---|---|
| Couleur des vergetures (anormale->normale) | -18% (tendance proche de la significative) | -26%* |
| Relief des vergetures (creuses/ boursouflées ->normales) | - 26%* | -16%* |

\* valeur statistiquement significative au temps 6 semaines, par rapport à l'évatuation initiale

5.4. Tolérance du cosmétique appréciée par le volontaire

- Manifestations cutanées ressenties

    . non        100%

    . pas de différence        100%

- Produit le mieux toléré

Aucune réaction d'irritation pathologique et significative d'une intolérance cutanée n'a été relevée. Les 9 volontaires ont également indiqué n'avoir observé aucune manifestation d'irritation et/ou d'inconfort au cours de l'essai.

6. Conclusion

En conclusion, les crèmes anti-vergetures des exemples 1 et 2, qui ne diffèrent entre elles que par l'actif anti-vergetures utilisé, appliquées pendant 6 semaines consécutives dans les conditions normales d'utilisation, chez 9 volontaires adultes de sexe féminin, ont permis d'obtenir un effet de régression des vergetures, mis en évidence par des méthodes instrumentales et sur la base de scores cliniques.
Cet effet s'est traduit:

- pour la crème de l'exemple 1 (utilisation du "Phytokine®") :

    . par une amélioration statistiquement significative des composantes de la tonicité et de la fermeté de la peau;
    . par une régression statistiquement significative de la largeur des vergetures (-17%) et de leur relief (-26%), avec une tendance non significative sur leur couleur (-18%);

- pour la crème de l'exemple 2 (utilisation du "Kollaren-CPP®") :

    . par une nette tendance, non significative, à l'amélioration des composantes de la tonicité et de la fermeté de la peau;
    . par une diminution statistiquement significative de la longueur des vergetures en comparaison à la crème de l'exemple 1;
    . par une amélioration statistiquement significative de la couleur (-26%) et du relief (-16%) des vergetures.

**REFERENCES BIBLIOGRAPHIQUES**

- Leveque J.L., Corcuff P. The Surface of the Skin - The Microrelief In Non Invasive Methods for the Quantification of Skin Functions: An Uptade on Methodology and Clinical Applications. Frosch P.J., Kligman A.M. Eds, Springer-Verlag, Berlin, New York, Paris 1993; 3-24.

- Wilhelm K.P., Cua A.B. and Maibach H.I. In vivo study on age-related elastic properties of Human skin. In "Noninvasive Methods for the Quantification of skin Functions: An Update on Methodology and clinical Applications".

**EP 1 119 344 B1**

Frosch P.J. and Kigman A.M. Ed., Springer-Verlag, 1993: 190-203.

**Revendications**

1.  Méthode de prévention et/ou de traitement cosmétique des vergetures de la peau, **caractérisée par le fait que** l'on applique sur les zones de la peau susceptibles de former ou comprenant des vergetures une composition comprenant, dans un véhicule approprié, au moins un agent anti-vergetures choisi dans le groupe constitué par les peptides du soja, les tripeptides constitués des acides aminés Glycine, Histidine et Lysine, et les mélanges de ces peptides.

2.  Méthode de prévention et/ou de traitement cosmétique des vergetures selon la revendication 1, **caractérisée par le fait que** l'on applique sur les zones de la peau susceptibles de former ou comprenant des vergetures une composition comprenant, dans un véhicule approprié, au moins un agent anti-vergetures choisi dans le groupe constitué par les peptides du soja fermentés, les tripeptides constitués des acides aminés Glycine, Histidine et Lysine, et les mélanges de ces peptides.

3.  Méthode de prévention et/ou de traitement cosmétique selon la revendication 1 ou 2, **caractérisée par le fait que** l'agent anti-vergetures est choisi dans le groupe constitué par le peptide Phytokine® du soja, le tripeptide KOLLAREN-CPP® et les mélanges de ces peptides.

4.  Méthode de prévention et/ou de traitement cosmétique selon l'une quelconque des revendications 1 à 3, **caractérisée par le fait que** la proportion en agent anti-vergetures est comprise entre environ 0,1 et environ 10 % en poids, par rapport au poids total de la composition.

5.  Méthode de prévention et/ou de traitement cosmétique selon l'une quelconque des revendications 1 à 4, **caractérisée par le fait que** la composition comprend en outre au moins un $\alpha$-hydroxy acide, en association avec l'agent anti-vergetures.

6.  Méthode de prévention et/ou de traitement cosmétique selon la revendication 5, **caractérisée par le fait que** l'$\alpha$-hydroxy acide est l'acide lactique.

7.  Méthode de prévention et/ou de traitement cosmétique selon la revendication 5 ou 6, **caractérisée par le fait que** la proportion en $\alpha$-hydroxy acide est comprise entre environ 0,1 et environ 20 % en poids, par rapport au poids total de la composition.

8.  Méthode de prévention et/ou de traitement cosmétique selon l'une quelconque des revendications précédentes, **caractérisée par le fait que** la composition comprend un agent anti-vergetures choisi dans le groupe constitué par le peptide du soja Phytokine®, le tripeptide KOLLAREN-CPP® et les mélanges de ces peptides, en association avec l'acide lactique.

9.  Méthode de prévention et/ou de traitement cosmétique selon l'une quelconque des revendications précédentes, **caractérisée par le fait que** la composition comprend en outre un composé pour régler le pH à une valeur comprise entre environ 2 et environ 4.

10. Méthode de prévention et/ou de traitement cosmétique selon l'une quelconque des revendications précédentes, **caractérisée par le fait que** la composition comprend en outre au moins un composé inhibiteur de substance P et du neuropeptide Y.

11. Méthode de prévention et/ou de traitement cosmétique selon la revendication 10, **caractérisée par le fait que** l'inhibiteur de substance P et du neuropeptide Y est l'extrait *Enteromorpha compressa.*

12. Méthode de prévention et/ou de traitement cosmétique selon la revendication 10 ou 11, **caractérisée par le fait que** la proportion en composé inhibiteur de substance P et du neuropeptide Y est comprise entre environ 0,1 et environ 5 % en poids, par rapport au poids total de la composition.

13. Méthode de prévention et/ou de traitement cosmétique selon l'une quelconque des revendications précédentes, **caractérisée par le fait que** la composition comprend un agent anti-vergetures choisi dans le groupe constitué

**11**

par le peptide du soja Phytokine®, le tripeptide KOLLAREN-CPP® et les mélanges de ces peptides, en association avec l'acide lactique et l'extrait Enteromorpha compressa.

14. Méthode de prévention et/ou de traitement cosmétique selon l'une quelconque des revendications précédentes, **caractérisée par le fait que** la composition comprend en outre au moins un composé choisi dans le groupe constitué par l'extrait de *Sophora japonica*, le lactate de méthylsilanol, le gluconate de cuivre, le gluconate de zinc et les mélanges de ces derniers.

15. Utilisation d'une composition telle que définie à l'une quelconque des revendications 1 à 14, pour la préparation d'un médicament de dermatologie destiné à la prévention et/ou au traitement des vergetures de la peau.

**Patentansprüche**

1. Verfahren zur Verhütung und/oder kosmetischen Behandlung von weißen Streifen der Haut, **dadurch gekennzeichnet, dass** man auf die Zonen der Haut, die dafür empfänglich sind, weiße Streifen zu bilden oder diese umfassen, eine Zusammensetzung aufträgt, die in einem geeigneten Vehikel mindestens ein Mittel gegen weiße Streifen umfasst, das aus der Gruppe ausgewählt ist, die aus Soja-Peptiden, Tripeptiden, die aus den Aminosäuren Glycin, Histidin und Lysin aufgebaut sind, und Mischungen dieser Peptide besteht.

2. Verfahren zur Verhütung und/oder kosmetischen Behandlung von weißen Streifen nach Anspruch 1, **dadurch gekennzeichnet, dass** man auf die Zonen der Haut, die dafür empfänglich sind, weiße Streifen zu bilden oder diese umfassen, eine Zusammensetzung aufträgt, die in einem geeigneten Vehikel mindestens ein Mittel gegen weiße Streifen umfasst, das aus der Gruppe ausgewählt ist, die aus fermentierten Soja-Peptiden, Tripeptiden, die aus den Aminosäuren Glycin, Histidin und Lysin aufgebaut sind, und Mischungen dieser Peptide besteht.

3. Verfahren zur Verhütung und/oder kosmetischen Behandlung nach Anspruch 1 oder 2, **dadurch gekennzeichnet, dass** das Mittel gegen weiße Streifen aus der Gruppe ausgewählt ist, die aus dem Soja-Peptid Phytokine®, dem Tripeptid KOLLAREN-CPP® und Mischungen dieser Peptide besteht.

4. Verfahren zur Verhütung und/oder kosmetischen Behandlung nach irgendeinem der Ansprüche 1 bis 3, **dadurch gekennzeichnet, dass** der Anteil an Mittel gegen weiße Streifen etwa 0,1 bis etwa 10 Gewichts-%, bezogen auf das Gesamtgewicht der Zusammensetzung, beträgt.

5. Verfahren zur Verhütung und/oder kosmetischen Behandlung nach irgendeinem der Ansprüche 1 bis 4, **dadurch gekennzeichnet, dass** die Zusammensetzung darüber hinaus mindestens eine $\alpha$-Hydroxysäure in Verbindung mit dem Mittel gegen weiße Streifen umfasst.

6. Verfahren zur Verhütung und/oder kosmetischen Behandlung nach Anspruch 5, **dadurch gekennzeichnet, dass** die $\alpha$-Hydroxysäure Milchsäure ist.

7. Verfahren zur Verhütung und/oder kosmetischen Behandlung nach Anspruch 5 oder 6, **dadurch gekennzeichnet, dass** der Anteil an $\alpha$-Hydroxysäure etwa 0,1 bis etwa 20 Gewichts-%, bezogen auf das Gesamtgewicht der Zusammensetzung, beträgt.

8. Verfahren zur Verhütung und/oder kosmetischen Behandlung nach irgendeinem der vorangehenden Ansprüche, **dadurch gekennzeichnet, dass** die Zusammensetzung ein Mittel gegen weiße Streifen, das aus der Gruppe ausgewählt ist, die aus dem Soja-Peptid Phytokine®, dem Tripeptid KOLLAREN-CPP® und Mischungen dieser Peptide besteht, in Assoziation mit Milchsäure umfasst.

9. Verfahren zur Verhütung und/oder kosmetischen Behandlung nach irgendeinem der vorangehenden Ansprüche, **dadurch gekennzeichnet, dass** die Zusammensetzung darüber hinaus eine Verbindung umfasst, um den pH bei einem Wert von etwa 2 bis etwa 4 zu regeln.

10. Verfahren zur Verhütung und/oder kosmetischen Behandlung nach irgendeinem der vorangehenden Ansprüche, **dadurch gekennzeichnet, dass** die Zusammensetzung darüber hinaus mindestens eine Inhibitor-Verbindung der Substanz P und des Neuropeptids Y umfasst.

**11.** Verfahren zur Verhütung und/oder kosmetischen Behandlung nach Anspruch 10, **dadurch gekennzeichnet, dass** der Inhibitor der Substanz P und des Neuropeptids Y der Extrakt *Enteromorpha compressa* ist.

**12.** Verfahren zur Verhütung und/oder kosmetischen Behandlung nach Anspruch 10 oder 11, **dadurch gekennzeichnet, dass** der Anteil an Inhibitor-Verbindung der Substanz P und des Neuropeptids Y etwa 0,1 bis etwa 15 Gewichts-%, bezogen auf das Gesamtgewicht der Zusammensetzung, beträgt.

**13.** Verfahren zur Verhütung und/oder kosmetischen Behandlung nach irgendeinem der vorangehenden Ansprüche, **dadurch gekennzeichnet, dass** die Zusammensetzung ein Mittel gegen weiße Streifen, das aus der Gruppe ausgewählt ist, die aus dem Soja-Peptid Phytokine®, dem Tripeptid KOLLAREN-CPP® und Mischungen dieser Peptide besteht, in Assoziation mit Milchsäure und dem Extrakt Enteromorpha compressa umfasst.

**14.** Verfahren zur Verhütung und/oder kosmetischen Behandlung nach irgendeinem der vorangehenden Ansprüche, **dadurch gekennzeichnet, dass** die Zusammensetzung darüber hinaus mindestens eine Verbindung umfasst, die aus der Gruppe ausgewählt ist, die aus dem Extrakt von *Sophora japonica*, Methylsilanollactat, Kupfergluconat, Zinkgluconat und Mischungen dieser Letztgenannten besteht.

**15.** Verwendung einer Zusammensetzung, wie in irgendeinem der Ansprüche 1 bis 14 definiert, für die Herstellung eines dermatologischen Medikaments, das zur Verhütung und/oder Behandlung von weißen Streifen der Haut bestimmt ist.

## Claims

**1.** Cosmetic method for preventing and/or treating skin stretchmarks, **characterized in that** a composition is applied to the areas of skin liable to form or comprising stretchmarks, this composition comprising, in a suitable vehicle, at least one anti-stretchmark agent chosen from the group consisting of soya peptides and tripeptides consisting of the amino acids glycine, histidine and lysine, and mixtures of these peptides.

**2.** Cosmetic method for preventing and/or treating stretchmarks according to Claim 1, **characterized in that** a composition is applied to the areas of skin liable to form or comprising stretchmarks, this composition comprising, in a suitable vehicle, at least one anti-stretchmark agent chosen from the group consisting of fermented soya peptides and tripeptides consisting of the amino acids glycine, histidine and lysine, and mixtures of these peptides.

**3.** Cosmetic prevention and/or treatment method according to Claim 1 or 2, **characterized in that** the anti-stretchmark agent is chosen from the group consisting of the soya peptide Phytokine® and the tripeptide Kollaren-CPP®, and mixtures of these peptides.

**4.** Cosmetic prevention and/or treatment method according to any one of Claims 1 to 3, **characterized in that** the proportion of anti-stretchmark agent is between about 0.1% and about 10% by weight relative to the total weight of the composition.

**5.** Cosmetic prevention and/or treatment method according to any one of Claims 1 to 4, **characterized in that** the composition also comprises at least one $\alpha$-hydroxy acid, in combination with the anti-stretchmark agent.

**6.** Cosmetic prevention and/or treatment method according to Claim 5, **characterized in that** the $\alpha$-hydroxy acid is lactic acid.

**7.** Cosmetic prevention and/or treatment method according to Claim 5 or 6, **characterized in that** the proportion of $\alpha$-hydroxy acid is between about 0.1% and about 20% by weight relative to the total weight of the composition.

**8.** Cosmetic prevention and/or treatment method according to any one of the preceding claims, **characterized in that** the composition comprises an anti-stretchmark agent chosen from the group consisting of the soya peptide Phytokine® and the tripeptide Kollaren-CPP® and mixtures of these peptides, in combination with lactic acid.

**9.** Cosmetic prevention and/or treatment method according to any one of the preceding claims, **characterized in that** the composition also comprises a compound for adjusting the pH to a value of between about 2 and about 4.

10. Cosmetic prevention and/or treatment method according to any one of the preceding claims, **characterized in that** the composition also comprises at least one substance-P and neuropeptide-Y inhibitor compound.

11. Cosmetic prevention and/or treatment method according to Claim 10, **characterized in that** the substance-P and neuropeptide-Y inhibitor is the extract of *Enteromorpha compressa.*

12. Cosmetic prevention and/or treatment method according to Claim 10 or 11, **characterized in that** the proportion of substance-P and neuropeptide-Y inhibitor compound is between about 0.1% and about 5% by weight relative to the total weight of the composition.

13. Cosmetic prevention and/or treatment method according to any one of the preceding claims, **characterized in that** the composition comprises an anti-stretchmark agent chosen from the group consisting of the soya peptide Phytokine® and the tripeptide Kollaren-CPP® and mixtures of these peptides, in combination with lactic acid and *Enteromorpha compressa* extract.

14. Cosmetic prevention and/or treatment method according to any one of the preceding claims, **characterized in that** the composition also comprises at least one compound chosen from the group consisting of extract of *Sophora japonica,* methylsilaryl lactate, copper gluconate and zinc gluconate, and mixtures of these compounds.

15. Use of a composition as defined in any one of Claims 1 to 14, to prepare a dermatological medicinal product for preventing and/or treating skin stretchmarks.